(19) Europäisches Patentamt
European Patent Office
Office européen des brevets

(11) Veröffentlichungsnummer : **0 015 456**
**B1**

(12) **EUROPÄISCHE PATENTSCHRIFT**

(45) Veröffentlichungstag der Patentschrift : **02.12.81**

(21) Anmeldenummer : **80100882.2**

(22) Anmeldetag : **22.02.80**

(51) Int. Cl.³ : **C 07 C131/00**, C 07 D233/56, C 07 D249/08

(54) **Omega-Halogen-acetophenon-oximether, Verfahren zu ihrer Herstellung sowie ihre Verwendung als Zwischenprodukte zur Herstellung von omega-Azolyl-acetophenon-oximethern.**

(30) Priorität : **01.03.79 DE 2907972**

(43) Veröffentlichungstag der Anmeldung : **17.09.80 (Patentblatt 80/19)**

(45) Bekanntmachung des Hinweises auf die Patenterteilung : **02.12.81 Patentblatt 81/48**

(84) Benannte Vertragsstaaten : **BE CH DE FR GB IT NL**

(56) Entgegenhaltungen : **Keine**

(73) Patentinhaber : **BAYER AG**
**Zentralbereich Patente, Marken und Lizenzen**
**D-5090 Leverkusen 1, Bayerwerk (DE)**

(72) Erfinder : **Krämer, Wolfgang, Dr.**
**Am Eckbusch 39/162**
**D-5600 Wuppertal 1 (DE)**

**EP 0 015 456 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

ω-Halogen-acetophenon-oximether, Verfahren zu ihrer Herstellung sowie ihre Verwendung als Zwischenprodukte zur Herstellung von ω-Azolyl-acetophenon-oximethern

Die vorliegende Erfindung betrifft neue ω-Halogen-acetophenon-oximether, ein Verfahren zur ihrer Herstellung und deren Verwendung als Zwischenprodukte zur Herstellung von weitgehend bekannten ω-Azolyl-acetophenon-oximethern mit fungizider und bakterizider Wirkung.

Es ist bereits bekannt geworden, daß man fungizid wirksame ω-Azolyl-acetophenon-oximether erhält, wenn man entsprechende ω-Azolyl-acetophenone mit Hydroxylamin in die ω-Azolyl-acetophenon-oxime überführt und letztere mit einem Halogenderivat umsetzt (vergleiche DE-OS 26 57 578 und DE-OS 27 23 942). Dieser Syntheseweg weist jedoch einige Nachteile auf.

Es wurden nun die neuen ω-Halogen-acetophenon-oximether der allgemeinen Formel

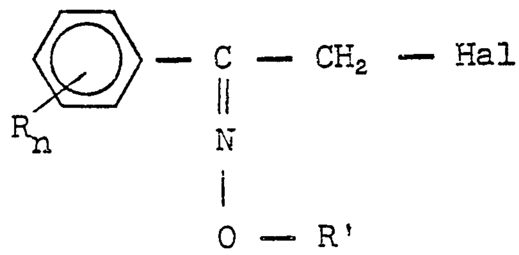

(I)

in welcher

R für Fluor, Chlor, Brom, Nitro und Cyano steht, ferner für Alkyl und Alkylsulfonyl mit jeweils 1 bis 4 Kohlenstoffatomen, für Alkoxy und Alkylthio mit 1 bis 2 Kohlenstoffatomen sowie für Halogen-alkyl mit bis zu 2 Kohlenstoff- und bis zu 3 Halogenatomen steht, außerdem für Phenyl oder Phenoxy steht, wobei diese beiden Reste substituiert sein können durch Halogen, Cyano, Nitro sowie Halogenalkyl mit bis zu 2 Kohlenstoff- und bis zu 3 Halogenatomen,

R' für Alkyl, Alkenyl und Alkinyl mit jeweils bis zu 4 Kohlenstoffatomen und für Cycloalkylalkyl mit 5 bis 7 Kohlenstoffatomen im Cycloalkylteil und 1 bis 2 Kohlenstoffatomen im Alkylteil steht, wobei der Cycloalkylteil gegebenenfalls durch Alkyl mit 1 bis 4 Kohlenstoffatomen substituiert sein kann, sowie für Benzyl oder Styryl steht, wobei die beiden genannten Reste substituiert sein können durch Halogen, Cyano, Nitro, Amino, Alkyl mit 1 bis 4 Kohlenstoffatomen, Phenyl, Phenoxy und Halogenalkyl mit bis zu 2 Kohlenstoff- und bis zu 3 Halogenatomen,

n für die Zahlen 1 oder 2 steht und

Hal für Chlor oder Brom steht,

gefunden.

Die Verbindungen der Formel (I) können in der syn- oder anti-Form vorliegen ; vorwiegend fallen sie als Gemische beider Formen an.

Man erhält die ω-Halogen-acetophenon-oximether der allgemeinen Formel (I), wenn man ω-Halogen-acetophenone der Formel

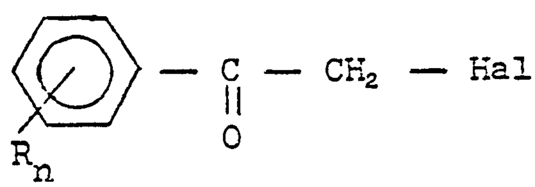

(II)

in welcher

Hal, R und n die oben angegebene Bedeutung haben,

mit substituierten Hydroxylaminen der Formel

$$H_2N — O — R' \qquad (III)$$

in welcher

R' die oben angegebene Bedeutung hat,

in Gegenwart eines Verdünnungsmittels umsetzt.

Die neuen Verbindungen der Formel (I) sind interessante Zwischenprodukte zur Herstellung von weitgehend bekannten ω-Azolyl-acetophenon-oximethern (vergleiche DE-OS 26 57 578 und DE-OS 27 23 942) der Formel

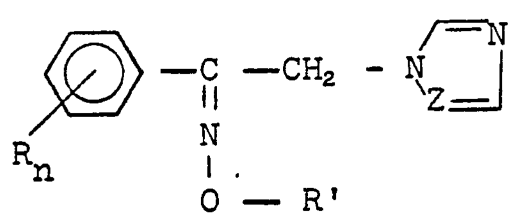

(IV)

in welcher

R, R' und n die oben angegebene Bedeutung haben und

Z für die CH-Gruppe oder ein Stickstoffatom steht.

Die aus den neuen Zwischenprodukten der Formel (I) erhältlichen bekannten Endprodukte der Formel (IV) sind Wirkstoffe mit fungizider, sowie auch bakterizider Wirkung und können z.B. als Pflanzenschutzmittel Verwendung finden. Die Endprodukte (IV) lassen sich — wie noch aufgezeigt wird — aus den neuen Zwischenprodukten (I) in einfacher Weise aufbauen und der neue Syntheseweg weist eine Reihe von Vorteilen in Relation zu dem vorbekannten Weg auf.

Die erfindungsgemäßen Stoffe sind durch die Formel (I) allgemein definiert.

Ganz besonders bevorzugt sind diejenigen ω-Halogen-acetophenon-oximeter der Formel (I), in denen *R* für Chlor, Brom, Phenyl, Chlorphenyl, Bromphenyl, Nitrophenyl, Phenoxy, Chlorphenoxy, Bromphenoxy oder Nitrophenoxy steht ; *n* für die Zahlen 1 oder 2 steht und *R'* für Methyl, Ethyl, n-Propyl, Isopropyl, n-Butyl, Isobutyl, sek.-Butyl oder tert.-Butyl ; Allyl, 2-Methylallyl ; Propargyl ; Cyclohexylmethyl, Methylcyclohexylmethyl, Benzyl, das gegebenenfalls ein- oder zweifach substituiert sein kann durch Chlor, Brom, Nitro, Methyl, Ethyl, Phenyl oder Phenoxy ; sowie für gegebenenfalls ein- oder zweifach durch Chlor substituiertes Styryl steht.

Im einzelnen seien die Verbindungen der folgenden Tabelle gennant :

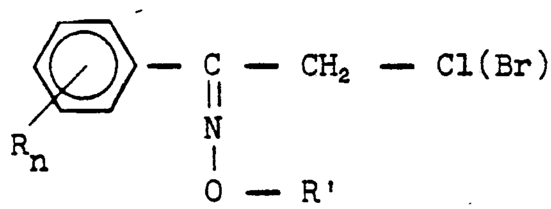

(Ia)

| $R_n$ | R' | $R_n$ | R' |
|---|---|---|---|
| 2,4—$Cl_2$ | 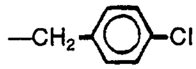 | 2,4—$Cl_2$ | $CH_3$ |
| 2,4—$Cl_2$ | 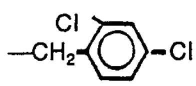 | 2,4—$Cl_2$ | $C_2H_5$ |
| 2,4—$Cl_2$ | 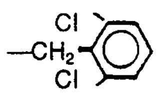 | 2,4—$Cl_2$ | $C_3H_7$ |
| 2,4—$Cl_2$ | 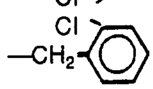 | 2,4—$Cl_2$ | $C_4H_9$ |
| 2,4—$Cl_2$ | 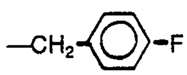 | 2,4—$Cl_2$ | $—CH_2—CH{=}CH_2$ |
| 2,4—$Cl_2$ | 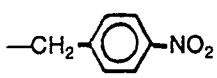 | 2,4—$Cl_2$ | $—CH_2—C{\equiv}CH$ |
| 2,4—$Cl_2$ | 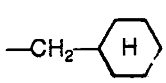 | | |
| 2,5—$Cl_2$ | 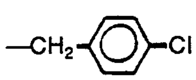 | 2,5—$Cl_2$ | $CH_3$ |
| 2,5—$Cl_2$ | 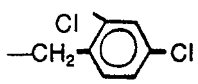 | 2,5—$Cl_2$ | $C_2H_5$ |
| 2,5—$Cl_2$ | 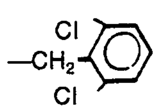 | 2,5—$Cl_2$ | $C_3H_7$ |
| 2,5—$Cl_2$ | 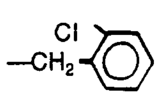 | 2,5—$Cl_2$ | $C_4H_9$ |
| 2,5—$Cl_2$ | 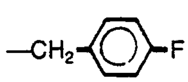 | 2,5—$Cl_2$ | $—CH_2—CH{=}CH_2$ |

| $R_n$ | R′ | $R_n$ | R′ |
|---|---|---|---|
| 2,5—$Cl_2$ | 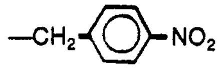 | 2,5—$Cl_2$ | $—CH_2—C{\equiv}CH$ |
| 2,5—$Cl_2$ | 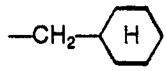 | | |
| 3,4—$Cl_2$ | 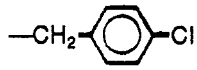 | 3,4—$Cl_2$ | $CH_3$ |
| 3,4—$Cl_2$ | 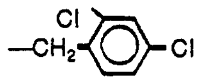 | 3,4—$Cl_2$ | $C_2H_5$ |
| 3,4—$Cl_2$ | 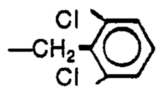 | 3,4—$Cl_2$ | $C_3H_7$ |
| 3,4—$Cl_2$ | 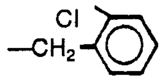 | 3,4—$Cl_2$ | $C_4H_9$ |
| 3,4—$Cl_2$ | 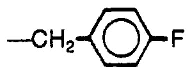 | 3,4—$Cl_2$ | $—CH_2—CH{=}CH_2$ |
| 3,4—$Cl_2$ | 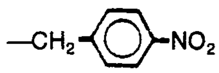 | 3,4—$Cl_2$ | $—CH_2—C{\equiv}CH_2$ |
| 3,4—$Cl_2$ | 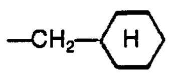 | | |
| 4—Cl | 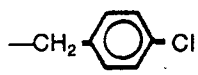 | 4—Cl | $CH_3$ |
| 4—Cl | 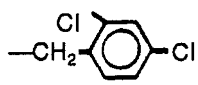 | 4—Cl | $C_2H_5$ |
| 4—Cl | 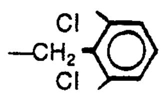 | 4—Cl | $C_3H_7$ |
| 4—Cl | 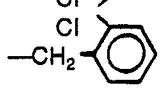 | 4—Cl | $C_4H_9$ |
| 4—Cl | 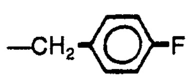 | 4—Cl | $—CH_2—CH{=}CH_2$ |
| 4—Cl | 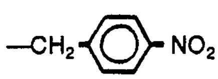 | 4—Cl | $—CH_2—C{\equiv}CH$ |
| 4—Cl | 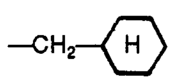 | | |
| 4—Br | 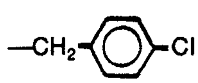 | 4—Br | $CH_3$ |
| 4—Br | 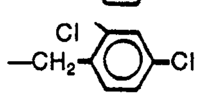 | 4—Br | $C_2H_5$ |
| 4—Br | 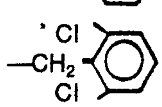 | 4—Br | $C_3H_7$ |
| 4—Br | 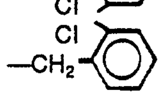 | 4—Br | $C_4H_9$ |
| 4—Br | 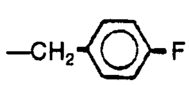 | 4—Br | $—CH_2—CH{=}CH_2$ |

| $R_n$ | R′ | $R_n$ | R′ |
|---|---|---|---|
| 4—Br | 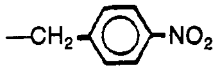 | 4—Br | $—CH_2—C≡CH_2$ |
| 4—Br | 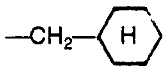 | | |
| 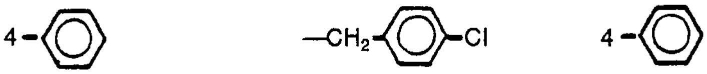 | | | $CH_3$ |
| 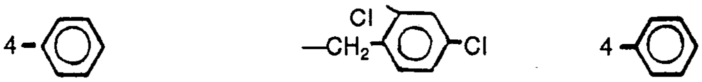 | | | $C_2H_5$ |
| 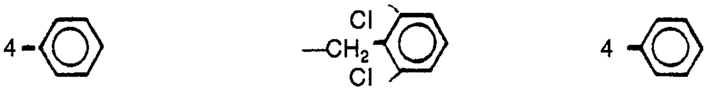 | | | $C_3H_7$ |
| 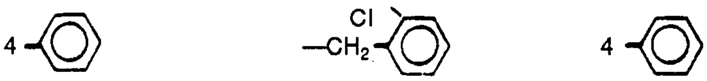 | | | $C_4H_9$ |
| 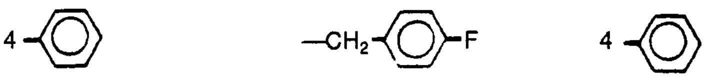 | | | $—CH_2—CH=CH_2$ |
| 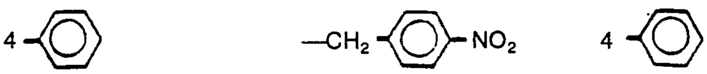 | | | $—CH_2—C≡CH$ |
| 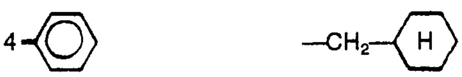 | | | |
| 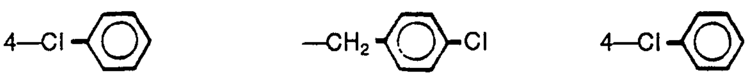 | | | $CH_3$ |
| 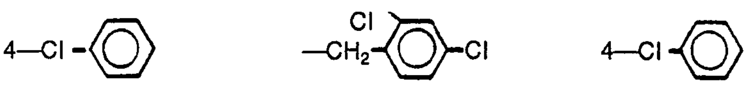 | | | $C_2H_5$ |
| 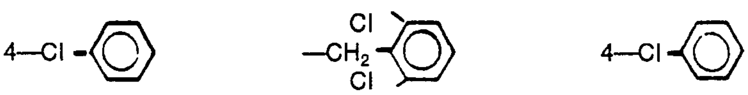 | | | $C_3H_7$ |
| 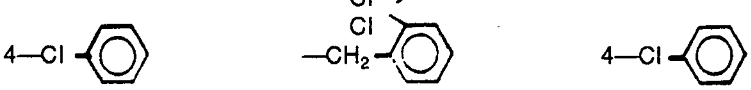 | | | $C_4H_9$ |
| 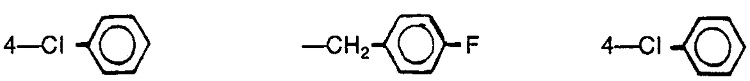 | | | $—CH_2—CH=CH_2$ |
| 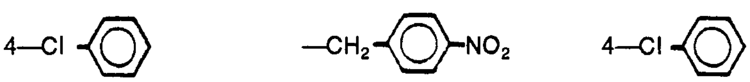 | | | $—CH_2—C≡CH_2$ |
| 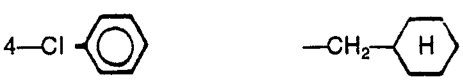 | | | |
| 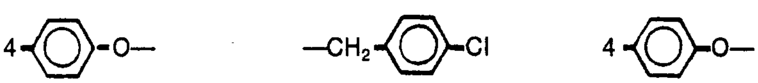 | | | $CH_3$ |
| 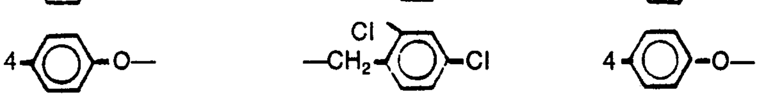 | | | $C_2H_5$ |
| 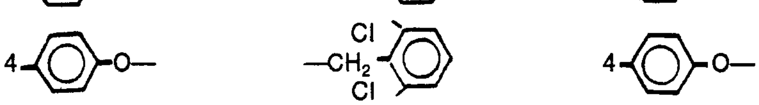 | | | $C_3H_7$ |
| 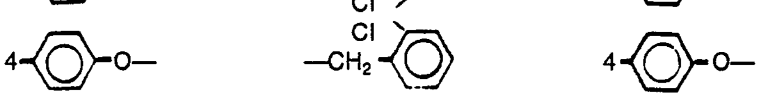 | | | $C_4H_9$ |
| 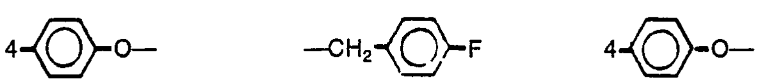 | | | $—CH_2—CH=CH_2$ |

| $R_n$ | R' | $R_n$ | R' |
|---|---|---|---|
| 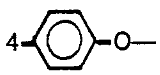 | 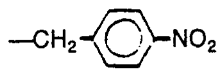 | 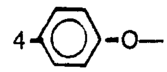 | $-CH_2-C{\equiv}CH$ |
| 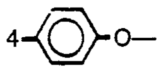 | 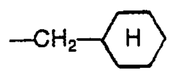 | | |

| $R_n$ | R' |
|---|---|
| 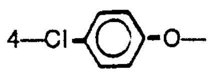 | $CH_3$ |
| 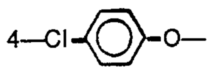 | $C_2H_5$ |
| 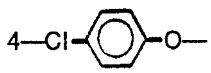 | $C_3H_7$ |
| 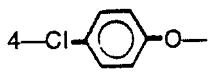 | $C_4H_9$ |
| 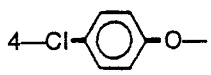 | $-CH_2-CH{=}CH_2$ |
| 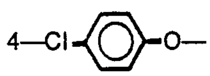 | $-CH_2-C{\equiv}CH$ |
| 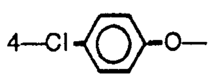 | 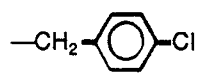 |
| 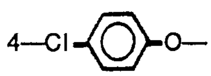 | 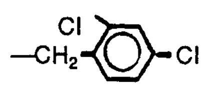 |
| 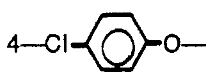 | 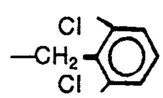 |
| 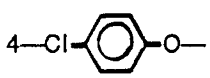 | 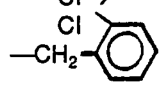 |
| 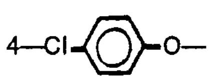 | 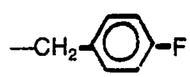 |
| 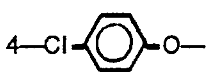 | 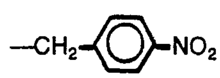 |
| 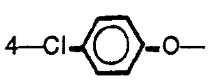 | 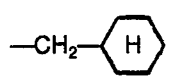 |

Verwendet man beispielsweise ω-Chlor-2,4-dichloracetophenon und O-methyl-hydroxylaminhydrochlorid als Ausgangsstoffe, so kann der Reaktionsablauf durch das folgende Formelschema wiedergegeben werden :

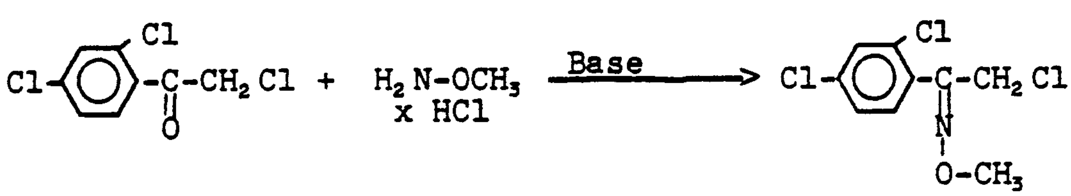

Die als Ausgangsstoffe für die Herstellung der Verbindungen der Formel I zu verwendenden ω-Halogen-acetophenone sind durch die Formel (II) allgemein definiert. In dieser Formel stehen *Hal, R* und *n* vorzugsweise für die Reste, die bereits im Zusammenhang mit der Beschreibung der erfindungsgemäßen Stoffe der Formel (I) vorzugsweise für diese Substituenten genannt wurden.

Die ω-Halogen-acetophenone sind bekannt (vergleiche Bulletin de la Société Chimique de France *1955,* Seiten 1 363-1 383). Sie lassen sich in allgemein bekannter Weise herstellen, indem man z.B. entsprechende Benzole in einer Friedel-Crafts-Reaktion mit Chlor- oder Bromacetylchlorid umsetzt oder entsprechende Acetophenone in üblicher Weise halogeniert, wobei vorzugsweise Sulfurylchlorid oder Brom als Halogenierungsmittel in Frage kommen.

Als Beispiele für ω-Halogen-acetophenone seien genannt :

ω-Chlor(Brom)-4-chloracetophenon
ω-Chlor(Brom)-2,4-dichloracetophenon
ω-Chlor(Brom)-3,4-dichloracetophenon
ω-Chlor(Brom)-4-bromacetophenon
ω-Chlor(Brom)-4-nitroacetophenon
ω-Chlor(Brom)-4-cyanacetophenon
ω-Chlor(Brom)-2-methylacetophenon
ω-Chlor(Brom)-2-methoxyacetophenon
ω-Chlor(Brom)-2-ethyl-4-chloracetophenon
ω-Chlor(Brom)-2-ethylthioacetophenon
ω-Chlor(Brom)-4-methylsulfonylacetophenon
ω-Chlor(Brom)-2,4,5-trichloracetophenon
ω-Chlor(Brom)-4-phenylacetophenon
ω-Chlor(Brom)-4-4'-chlorphenylacetophenon
ω-Chlor(Brom)-4-4'-bromphenylacetophenon
ω-Chlor(Brom)-4-4'-nitrophenylacetophenon
ω-Chlor(Brom)-4-phenoxyacetophenon
ω-Chlor(Brom)-4-4'-chlorphenoxyacetophenon
ω-Chlor(Brom)-4-4'-bromphenoxyacetophenon
ω-Chlor(Brom)-4-4'-nitrophenoxyacetophenon

Die weiterhin als Ausgangsstoffe zu verwendenden substituierten Hydroxylamine sind durch die Formel (III) allgemein definiert. In dieser Formel steht *R'* vorzugsweise für die Reste, die bereits im Zusammenhang mit der Beschreibung der erfindungsgemäßen Stoffe der Formel (I) vorzugsweise für diesen Substituenten genannt wurden.

Die substituierten Hydroxylamine der Formel (III) sind allgemein bekannte Verbindungen der organischen Chemie. Als Beispiele seien genannt :

O-Methyl-hydroxylamin
O-Ethyl-hydroxylamin
O-n-Propyl-hydroxylamin
O-Isopropyl-hydroxylamin
O-n-Butyl-hydroxylamin
O-tert.-Butyl-Hydroxylamin
O-Allyl-hydroxylamin
O-2-Methylallyl-hydroxylamin
O-Propargyl-hydroxylamin
O-4-Chlorbenzyl-hydroxylamin
O-2-Chlorbenzyl-hydroxylamin
O-2,4-Dichlorbenzyl-hydroxylamin
O-3,4-Dichlorbenzyl-hydroxylamin
O-2,6-Dichlorbenzyl-hydroxylamin
O-2,5-Dichlorbenzyl-hydroxylamin
O-4-Brombenzyl-hydroxylamin
O-4-Fluorbenzyl-hydroxylamin
O-4-Nitrobenzyl-hydroxylamin
O-4-Methylbenzyl-hydroxylamin
O-4-Phenoxybenzyl-hydroxylamin
O-3-Phenoxybenzyl-hydroxylamin
O-4-Phenylbenzyl-hydroxylamin
O-Styryl-hydroxylamin
O-2,4-Dichlorstyryl-hydroxylamin
O-Cyclohexylmethyl-hydroxylamin
O-Methylcyclohexylmethyl-hydroxylamin

Für die erfindungsgemäße Umsetzung zur Herstellung der Verbindungen der Formel I kommen als Verdünnungsmittel vorzugsweise Alkohole bzw. wäßrige Alkohole in Frage.

Die Reaktionstemperaturen können in einem größeren Bereich variiert werden. Im allgemeinen arbeitet man zwischen 20 und 120 °C, vorzugsweise zwischen 50 und 100 °C.

Bei der Durchführung des erfindungsgemäßen Verfahrens setzt man auf 1 Mol Acetophenon der Formel (II) vorzugsweise 1 bis 1,2 Mol Hydroxylamin der Formel (III) ein. Die Isolierung der Verbindungen der Formel (I) erfolgt nach üblichen Methoden.

Nach einer bevorzugten Ausführungsform werden die Hydroxylamine der Formel (III) in Form ihrer Salze, insbesondere als Hydrochloride, gegebenenfalls in Gegenwart eines Säurebindemittels, wie beispielsweise Natriumacetat, eingesetzt (vergleiche auch die Herstellungsbeispiele).

Wie schon erwähnt, können die neuen Verbindungen der Formel I in vorteilhafter Weise dazu Verwendung finden, die bekannten Wirkstoffe der Formel IV herzustellen.

Es ist gemäß dem Stande der Technik bekannt, daß man fungizid wirksame ω-Azolyl-acetophenon-oximether erhält, wenn man in einer ersten Stufe ω-Halogen-acetophenone mit Azolen (1,2,4-Triazol oder Imidazol) in Gegenwart eines inerten organischen Lösungsmittels und in Gegenwart eines Säurebinders bei Temperaturen zwischen 20 und 120 °C umsetzt ; die entstehenden ω-Azolyl-acetophenone in einer zweiten Stufe mit Hydroxylamin in Gegenwart eines Lösungsmittels, vorzugsweise Alkohole, bei 50 bis 80 °C umsetzt, wobei das Hydroxylamin vorzugsweise als Hydrochlorid in Gegenwart eines Säurebindemittels eingesetzt wird, und die entstehenden ω-Azolyl-acetophenon-oxime in einer dritten Stufe in Gegenwart eines inerten organischen Lösungsmittels und gegebenenfalls in Gegenwart einer starken Base bei Temperaturen zwischen 60 und 100 °C mit einem Halogenderivat gemäß dem folgenden Reaktionsschema zu den entsprechenden Ethern umsetzt (vergleiche DE-OS 26 57 578 und DE-OS 27 23 942) :

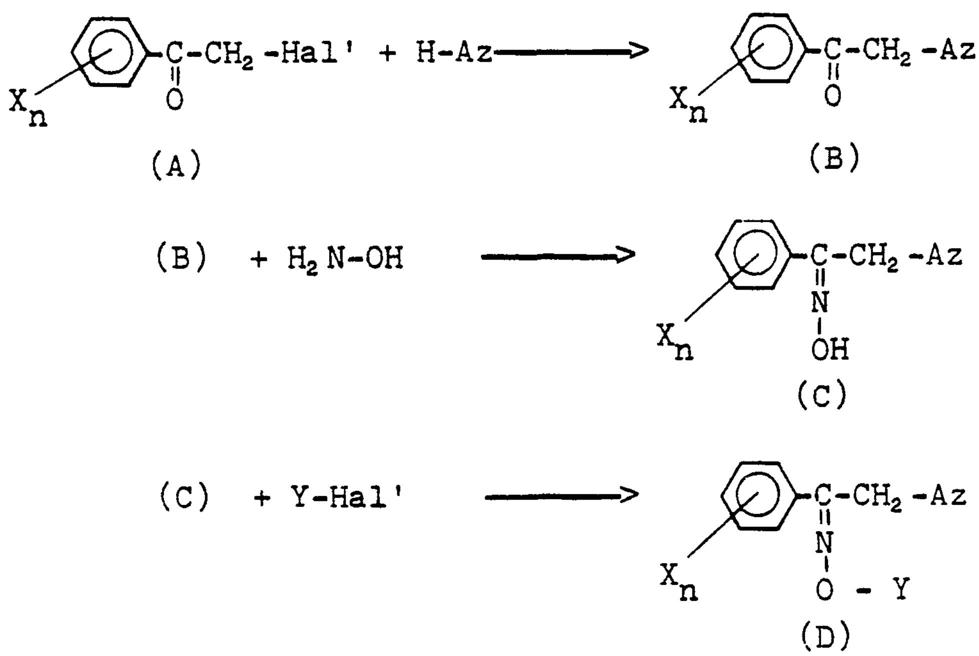


Hierin bedeuten :

Az = 1,2,4-Triazolyl, Imidazolyl ;

X = Halogen, Alkyl, Alkoxy, Nitro, gegebenenfalls substituiertes Phenyl, gegebenenfalls substituiertes Phenoxy ;

Hal' = Halogen ;

Y = Alkyl, Alkenyl, Alkinyl, Cycloalkyl, gegebenenfalls substituiertes Aralkyl ;

n = 0, 1, 2, 3.

Dieses bekannte Verfahren hat den Nachteil, daß es in nicht befriedigender Gesamtausbeute an ω-Azolyl-acetophenon-oximethern der Formel D abläuft.

Wie nun weiterhin gefunden wurde, kann man die weitgehend bekannten ω-Azolyl-acetophenon-oximether der allgemeinen Formel

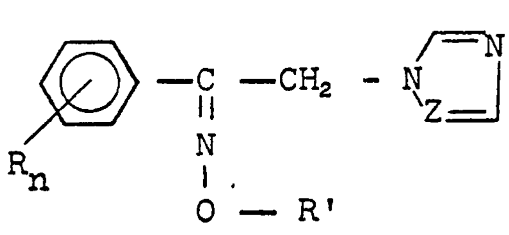

(IV)

in welcher R, R', Z und n die eingangs angegebene Bedeutung besitzen, in besonders vorteilhafter Weise dann erhalten, wenn man die neuen ω-Halogen-acetophenon-oximether (erfindungsgemäße Zwischenprodukte) der allgemeinen Formel

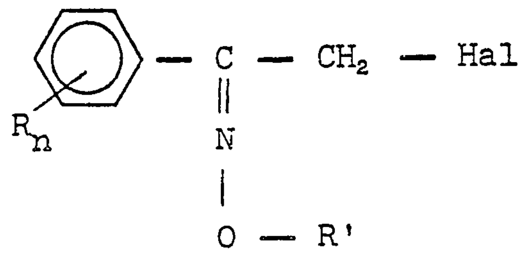

in welcher
Hal, R, R' und n die eingangs angegebene Bedeutung haben,
mit Azolen der Formel

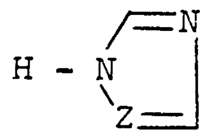

in welcher
Z die eingangs angegebene Bedeutung hat,
in Gegenwart eines Säurebindemittels und in Gegenwart eines Verdünnungsmittels umsetzt.

Es ist als ausgesprochen überraschend zu bezeichnen, daß auf dem Wege über die neuen Zwischenprodukte der Formel I die Endprodukte in gewünschter hoher Ausbeute und Reinheit erhalten werden, womit sich dieses Verfahren gegenüber dem aus dem Stand der Technik bekannten Syntheseweg als vorteilhafter erweist.

Verwendet man beispielsweise ω-Chlor-2,4-dichloracetophenon-oxim-O-methyl-ether und 1,2,4-Triazol als Ausgangsstoffe, so kann der Reaktionsablauf durch das folgende Formelschema wiedergegeben werden :

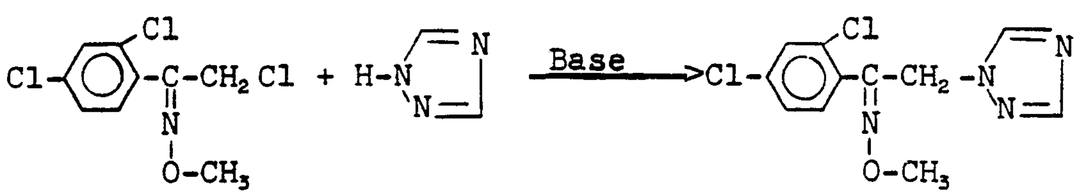


Die als Ausgangsstoffe für die Herstellung der Endprodukte der Formel IV zu verwendenden Azole sind durch die Formel (V) allgemein definiert. In dieser Formel steht *Z* vorzugsweise für die CH-Gruppe oder ein Stickstoffatom.

Die Azole der Formel (V) sind allgemein bekannte Verbindungen der organischen Chemie.

Zur Herstellung der Endprodukte der Formel IV ist noch im einzelnen zu vermerken :

Als Verdünnungsmittel kommen vorzugsweise inerte organische Lösungsmittel in Frage. Hierzu gehören Ketone, wie Aceton und Methyläthylketon ; Nitrile, wie Acetonitril ; Alkohole, wie Ethanol ; Ether, wie Tetrahydrofuran oder Dioxan ; aromatische Kohlenwasserstoffe, wie Toluol und Benzol ; Formamide, wie Dimethylformamid ; sowie halogenierte Kohlenwasserstoffe, wie Methylenchlorid, Tetrachlorkohlenstoff oder Chloroform.

Die Umsetzung wird in Gegenwart eines Säurebinders vorgenommen. Man kann alle üblicherweise verwendbaren anorganischen oder organischen Säurebinder zugeben, wie Alkalicarbonate, z.B. Natriumcarbonat un Kaliumcarbonat ; oder wie niedere tertiäre Alkylamine, Cycloalkylamine oder Aralkylamine, z.B. Triethylamin, N,N-Dimethylcyclohexylamin und N,N-Dimethylbenzylamin. Ebenfalls möglich ist ein entsprechender Überschuß an Azol der Formel (V).

Die Reaktionstemperaturen können in einem größeren Bereich variiert werden. Im allgemeinen arbeitet man zwischen 0 bis 150 °C, vorzugsweise bei 60 bis 120 °C.

Zur Durchführung der Umsetzung setzt man auf 1 Mol der Verbindungen der Formel (IV) vorzugsweise 1 bis 2 Mol Azol und 1 bis 2 Mol Säurebinder ein. Die Isolierung der Verbindungen der Formel (I) erfolgt nach üblichen Methoden. Da diese meistens in Form von Ölen anfallen, werden sie vorzugsweise als Salze, wie insbesondere als Hydrochloride oder Nitrate, isoliert.

Gemäß einer besonderen Ausführungsform kann auch so vorgegangen werden, daß man zunächst

die erfindungsgemäßen Zwischenprodukte der Formel I herstellt und ohne deren Isolierung und ohne Lösungsmittelwechsel die weitere Umsetzung durchführt und die ω-Azolyl-acetephenon-oximether (Endprodukte der Formel IV) im Rahmen eines « Eintopfverfahrens » in einem Arbeitsgang erhält.

Die ω-Azolyl-Acetophenon-oximether der Formel IV zeichnen sich bekanntermaßen durch eine sehr gute fungizide Wirksamkeit aus (vgl. DE-OS 26 57 578 und DE-OS 27 23 942), sowie auch die veröffentliche europäische Patentanmeldung Nr. 0 004 917).

In den Herstellungsbeispielen wird der neue Weg zur Herstellung der bekannten Verbindungen der Formel IV über die neuen Zwischenprodukte der Formel I aufgezeigt und den bekannten Verfahren gegenübergestellt.

## Herstellungsbeispiele

A) Neue Zwischenprodukte der Formel I

### Beispiel A 1 :

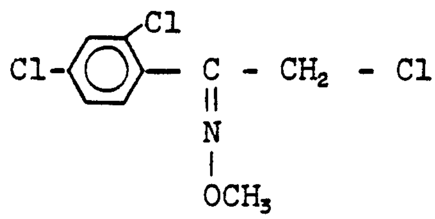

22,3 g (0,1 Mol) ω-Chlor-2,4-dichloracetophenon werden mit 9,2 g (0,11 Mol) O-Methyl-hydroxylaminhydrochlorid und 8,2 g (0,1 Mol) Natriumacetat in 200 ml Ethanol 20 Stunden unter Rückfluß erhitzt. Das Lösungsmittel wird danach im Vakuum abdestilliert und der Rückstand in 100 ml Methylenchlorid aufgenommen. Man wäscht einmal mit 200 ml und zweimal mit je 100 ml Wasser, trocknet über Natriumsulfat und engt durch Abdestillieren des Lösungsmittels im Vakuum ein. Man erhält 25 g (99,6 % der Theorie) ω-Chlor-2,4-dichloracetophenon-oxim-O-methyl-ether als Oel mit einem Brechungsindex $n_D^{20} = 1,565\,2$.

In entsprechender Weise werden die folgenden Verbindungen der allgemeinen Formel

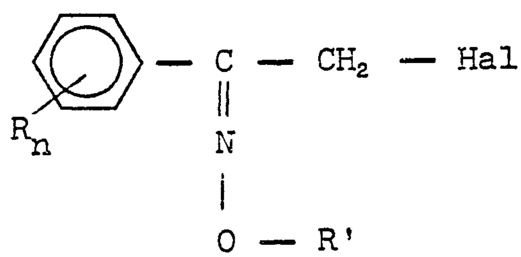

(I)

erhalten :

| Bsp. Nr. | $R_n$ | R' | Hal | Physikalische Konstante |
|---|---|---|---|---|
| A2 | 2,4—$Cl_2$ | 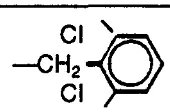 | Cl | $n_D^{20}$=1,590 1 |
| A3 | 2,4—$Cl_2$ | 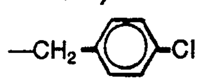 | Cl | $n_D^{23}$=1,587 9 |

B) Endprodukte der Formel IV

### Beispiel B 1 :

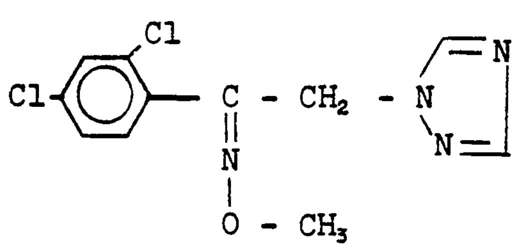

Erfindungsgemäße Darstellung mit Isolierung des Zwischenproduktes :

Es wird zunächst der ω-Chlor-2,4-dichloracetophenon-oxim-O-methyl-ether gemäß Beispiel A 1 hergestellt (siehe oben). Sodann wird wie folgt verfahren :

12,5 g (0,05 Mol) ω-Chlor-2,4-dichloracetophenon-oxim-O-methyl-ether werden in je 10 ml Dimethylformamid gelöst und zu 4 g (0,055 Mol) 1,2,4-Triazol sowie 7 g (0,05 Mol) Kaliumcarbonat in 50 ml Dimethylformamid getropft. Nach dem Zutropfen läßt man 20 Stunden bei 70 °C rühren und rührt die Reaktionsmischung nach dem Abkühlen in 200 ml Wasser ein. Danach wird mit 200 ml Methylenchlorid extrahiert, die organische Phase wird achtmal mit je 100 ml Wasser gewaschen, über Natriumsulfat getrocknet und durch Abdestillieren des Lösungsmittels im Vakuum eingeengt. Man erhält 12 g (84,5 % der Theorie) ω-(1,2,4-Triazol-1-yl)-2,4-dichloracetophenon-oxim-O-methylether als Oel (was einer Gesamtausbeute von 84,2 % der Theorie entspricht), das durch Behandlung mit Salpetersäure in Chloroform in das entsprechende Nitrat vom Zersetzungspunkt 90-92 °C überführt werden kann.

Vergleichsbeispiel :

(Darstellung nach vorbekanntem Verfahren)

1. Stufe :

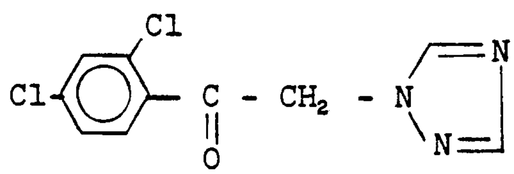

269 g (1 Mol) ω-Brom-2,4-dichloracetophenon werden in 250 ml Acetonitril gelöst. Diese Lösung tropft man zu einer unter Rückfluß siedenden Suspension von 69 g (1 Mol) 1,2,4-Triazol und 150 g Kaliumcarbonat in 2 000 ml Acetonitril. Nach 20-stündigem Erhitzen unter Rückfluß wird die erkaltete Suspension filtriert, das Filtrat vom Lösungsmittel befreit und der Rückstand mit Essigester aufgenommen, mit Wasser gewaschen, über Natriumsulfat getrocknet und von Lösungsmittel befreit. Der Essigester-Rückstand kristallisiert beim Versetzen mit Isopropanol aus. Nach dem Umkristallisieren aus Ligroin/Isopropanol erhält man 154 g (60 % der Theorie) ω-(1,2,4-Triazol-1-yl)-2,4-dichloracetophenon vom Schmelzpunkt 117 °C.

2. Stufe :

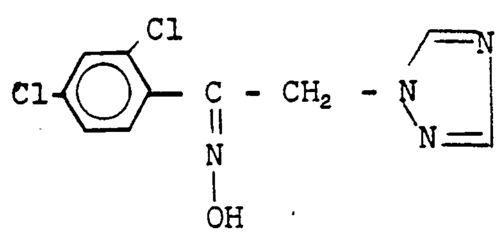

106,8 g (0,44 Mol) 1-(2,4-Dichlorphenyl)-2-(1,2,4-triazol-1-yl)-ethan-1-on werden in 780 ml Ethanol gelöst, mit 48 g Hydroxylammoniumhydrochlorid versetzt und 5 Stunden unter Rückfluß erhitzt. Danach wird das Reaktionsgemisch mit 1 000 ml Wasser versetzt und filtriert. Man erhält 51 g (45 % der Theorie) ω-(1,2,4-Triazol-1-yl)-2,4-dichloracetophenon-oxim vom Schmelzpunkt 165-170 °C.

Endstufe :

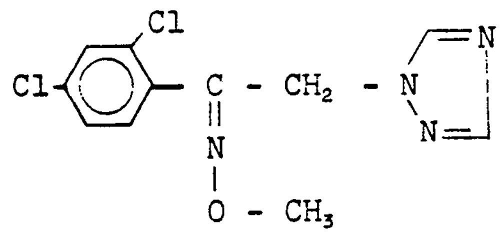

27,1 g (0,1 Mol) ω-(1,2,4-Triazol-1-yl)-2,4-dichloracetophenon-oxim werden in 300 ml Toluol, 300 ml konzentrierter Natronlauge und 2 ml Benzyl-dimethyl-ammonium-chlorid suspendiert, 28,4 g (0,2 Mol) Methyljodid hinzugegeben und 15 Stunden bei 40 °C gerührt. Danach läßt man abkühlen, trennt die organische Phase ab, wäscht sie dreimal mit je 200 ml gesättigter Natriumchloridlösung, trocknet über

Natriumsulfat und engt durch Abdestillieren des Lösungsmittels ein. Das zurückbleibende Oel enthält neben dem gewünschten Oximether auch ω-(1,2,4-Triazol-1-yl)-2,4-dichloracetophenon, das durch Verseifung des eingesetzten Oxims entsteht. Zur Reinigung wird der ölige Rückstand in 100 ml Chloroform gelöst und unter Eiskühlung mit 3 ml konzentrierter Salpetersäure versetzt. Nach Zugabe von 100 ml Ether fallen 4,2 g Nitrat von ω-(1,2,4-Triazol-1-yl)-2,4-dichloracetophenon-oxim-O-methyl-ether als reines Stereoisomeres vom Schmelzpunkt 111 bis 114 °C (Zersetzung) aus. Nach Abdestillieren des Lösungsmittels im Vakuum und Umkristallisieren des Rückstands aus Essigester werden weitere 3,2 g Nitrat von ω-(1,2,4-Triazol-1-yl)-2,4-dichloracetophenon-oxim-O-methyl-ether als Isomerengemisch vom Schmelzpunkt 90-92 °C erhalten, womit insgesamt 7,4 g (17 % der Theorie) ω-(1,2,4-Triazol-1-yl)-2,4-dichloracetophenon-oxim-O-methyl-ether erhalten werden. Das entspricht einer Gesamtausbeute über alle Stufen von 4,6 % der Theorie.

Beispiel B 2 :

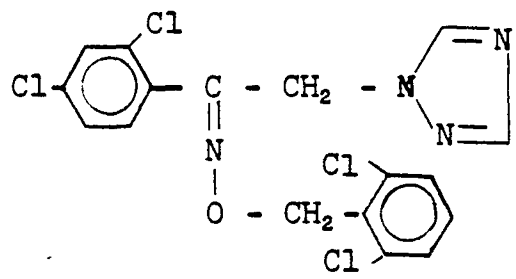

Erfindungsgemäße Darstellung, jedoch ohne Isolierung des Zwischenproduktes :
8,9 g (0,04 Mol) ω-Chlor-2,4-dichloracetophenon werden mit 8,6 g (0,04 Mol) 0-2,6-Dichlorbenzyl-hydroxylamin-hydrochlorid und 3,3 g (0,04 Mol) Natriumacetat in 100 ml Ethanol 15 Stunden unter Rückfluß erhitzt. Zu diesem Reaktionsgemisch werden 2,8 g (0,04 Mol) 1,2,4-Triazol und 5,6 g (0,04 Mol) Kaliumcarbonat gegeben und weitere 20 Stunden unter Rückfluß erhitzt. Nach dem Erkalten wird filtriert und das Filtrat eingeengt. Der Rückstand wird in 250 ml Methylenchlorid aufgenommen, fünfmal mit je 200 ml Wasser gewaschen, über Natriumsulfat getrocknet und eingeengt. Man erhält 15,4 g (89,5 % der Theorie) ω-(1,2,4-Triazol-1-yl)-2,4-dichloracetophenon-oxim-O-2,6-dichlorbenzyl-ether als Oel. Dieses wird zur Salzbildung in Aceton gelöst. Man filtriert eine Lösung von 5,4 g 1,5-Naphthalindisulfonsäure in 25 ml Aceton zu, verrührt 10 Minuten bei Raumtemperatur und saugt den Niederschlag ab. Man erhält 14,8 g (72 % der Theorie — bezogen auf eingesetzte Base) ω-(1,2,4-Triazol-1-yl)-2,4-dichloracetophenon-oxim-2,6-dichlorbenzyl-ether-1,5-naphthalindisulfonat vom Schmelzpunkt 245-252 °C (Zers.).

In entsprechender Weise werden die Endprodukte der allgemeinen Formel

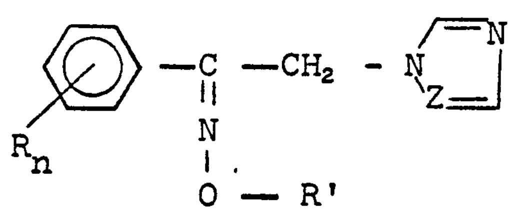

(IV)

erhalten :

| Bsp. Nr. | $R_n$ | R' | Z | Schmelzpunkt (°C) |
|---|---|---|---|---|
| B 3 | 2,4—$Cl_2$ | 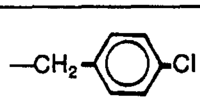 | N | 130-35 (Zers.) ($xHNO_3$) |
| B 4 | 2,4—$Cl_2$ | 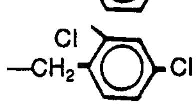 | N | 135-137 (Zers.) ($xHNO_3$) |
| B 5 | 2,4—$Cl_2$ | $C_2H_5$ | N | 65-72 |
| B 6 | 2,4—$Cl_2$ | $C_3H_7$ | N | 108-12 |
| B 7 | 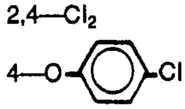 | $C_3H_7$ | N | 154-56 ($xHNO_3$) |

| Bsp. Nr. | $R_n$ | R' | Z | Schmelzpunkt (°C) |
|---|---|---|---|---|
| B 8 | 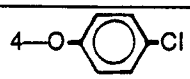 | $C_4H_9$ | N | 53-55 |
| B 9 | 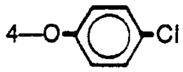 | 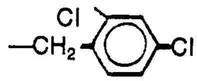 | N | 110-18 |
| B 10 | 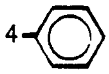 | 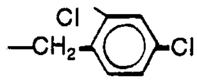 | N | 129-30 |
| B 11 | 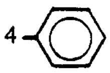 | $C_4H_9$ | N | 129-30 |
| B 12 | 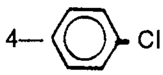 | 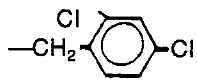 | N | 166 |
| B 13 | 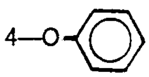 | $C_4H_9$ 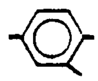 | N | 142-44 ($xHNO_3$) |
| B 14 | 2,5—$Cl_2$ | 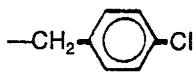 | N | 158-60 (xHCl) |
| B 15 | 3,4—$Cl_2$ | 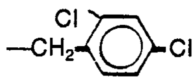 | N | 120-21 |
| B 16 | 2,4—$Cl_2$ | $—CH_2—CH{=}CH_2$ | N | 115 (Zers.) ($xHNO_3$) |
| B 17 | 2,4—$Cl_2$ | $—CH_2—CH{\equiv}CH$ | N | 108 (Zers.) ($xHNO_3$) |
| B 18 | 4—Br | $CH_3$ | N | 157-59 (xHCl) |
| B 19 | 4—Br | 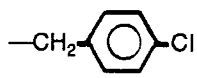 | N | 167-69 (xHCl) |
| B 20 | 4—Br | 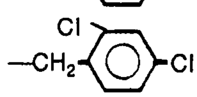 | N | 168-70 (xHCl) |
| B 21 | 4—Cl | $CH_3$ | N | 156-58 (xHCl) |
| B 22 | 4—Cl | 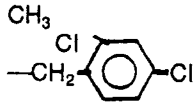 | N | 91 |
| B 23 | 4—Cl | 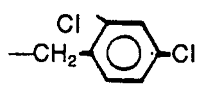 | CH | 127 (xHCl) |
| B 24 | 2,4—$Cl_2$ | 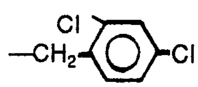 | CH | 137-38 ($xHNO_3$) |
| B 25 | 2,4—$Cl_2$ | 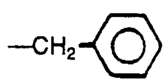 | CH | 119-22 ($xHNO_3$) |
| B 26 | 2,4—$Cl_2$ | 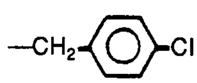 | CH | 121-26 ($xHNO_3$) |
| B 27 | 4—Cl | 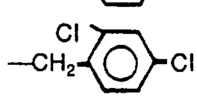 | CH | 142-47 ($xHNO_3$) |
| B 28 | 2,4—$(CH_3)_2$ | 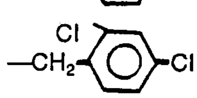 | CH | 151-54 ($xHNO_3$) |
| B 29 | 4—Br | 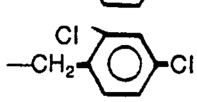 | CH | 152-54 ($xHNO_3$) |
| B 30 | 4—F | 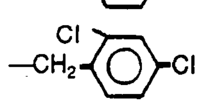 | CH | 124-26 ($xHNO_3$) |
| B 31 | 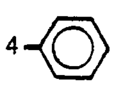 | 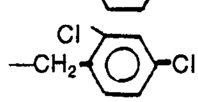 | CH | 171-77 ($xHNO_3$) |

| Bsp. Nr. | $R_n$ | R' | Z | Schmelzpunkt (°C) |
|---|---|---|---|---|
| B 32 | 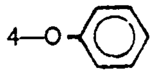 | 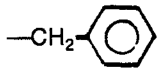 | CH | 93-100 ($xHNO_3$) |
| B 33 | 4—Cl | 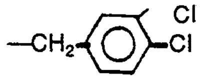 | CH | 152-55 ($xHNO_3$) |
| B 34 | 4—Cl | 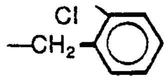 | CH | 116-18 ($xHNO_3$) |
| B 35 | 4—Cl | 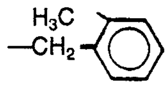 | CH | 167-69 ($xHNO_3$) |
| B 36 | 4—Cl | 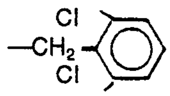 | CH | 180 ($xHNO_3$) |
| B 37 | 4—Cl | 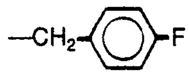 | CH | 170 ($xHNO_3$) |
| B 38 | 4—Cl | 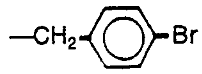 | CH | 146 ($xHNO_3$) |
| B 39 | 4—Br | 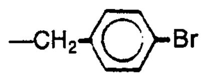 | CH | 167 ($xHNO_3$) |
| B 40 | 2,4—$Cl_2$ | 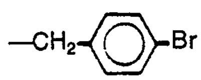 | CH | 162-63 ($xHNO_3$) |
| B 41 | 4—Br | 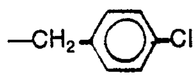 | CH | 169-70 ($xHNO_3$) |
| B 42 | 4—J | 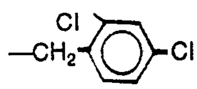 | CH | 163-64 ($xHNO_3$) |
| B 43 | 4—Cl | 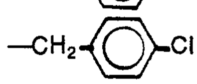 | CH | 157-58 ($xHNO_3$) |
| B 44 | 4—Cl | $C_2H_5$ | CH | 117-119 |

**Ansprüche**

1. ω-Halogen-acetophenon-oximether der allgemeinen Formel

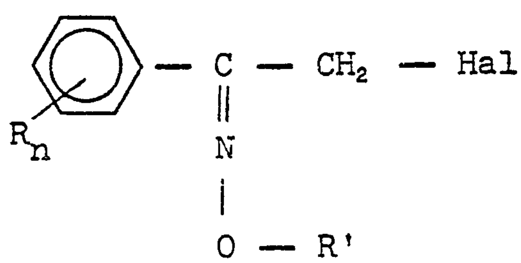

(I)

in welcher

R Fluor, Chlor, Brom, Nitro und Cyano steht, ferner für Alkyl und Alkylsulfonyl mit jeweils 1 bis 4 Kohlenstoffatomen, für Alkoxy und Alkylthio mit 1 bis 2 Kohlenstoffatomen sowie für Halogenalkyl mit bis zu 2 Kohlenstoff- und bis zu 3 Halogenatomen steht, außerdem für Phenyl oder Phenoxy steht, wobei diese beiden Reste substituiert sein können durch Halogen, Cyano, Nitro sowie Halogenalkyl mit bis zu 2 Kohlenstoff- und bis zu 3 Halogenatomen,

R' für Alkyl, Alkenyl und Alkinyl mit jeweils bis zu 4 Kohlenstoffatomen und für Cycloalkylalkyl mit 5 bis 7 Kohlenstoffatomen im Cycloalkylteil und 1 bis 2 Kohlenstoffatomen im Alkylteil steht, wobei der Cycloalkylteil gegebenenfalls durch Alkyl mit 1 bis 4 Kohlenstoffatomen substituiert sein kann, sowie für Benzyl oder Styryl steht, wobei die beiden genannten Reste substituiert sein können durch Halogen,

Cyano, Nitro, Amino, Alkyl mit 1 bis 4 Kohlenstoffatomen, Phenyl, Phenoxy und Halogenalkyl mit bis zu 2 Kohlenstoff- und bis zu 3 Halogenatomen,

n für die Zahlen 1 oder 2 steht und

Hal für Chlor oder Brom steht.

2. Verfahren zur Herstellung von ω-Halogen-acetophenon-oximethern der Formel I in Anspruch 1, dadurch gekennzeichnet, daß man ω-Halogen-acetophenone der Formel

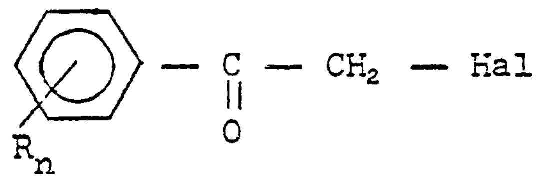
(II)

in welcher

Hal, R und n die in Anspruch 1 angegebene Bedeutung haben,

mit substituiert Hydroxylaminen der Formel

$$H_2N-O-R' \qquad (III)$$

in welcher

R' die in Anspruch 1 angegebene Bedeutung hat,

in Gegenwart eines Verdünnungsmittels umsetzt.

3. Verwendung von ω-Halogen-acetophenon-oximethern der Formel I in Anspruch 1 als Zwischenprodukte zur Herstellung von weitgehend bekannten ω-Azolyl-acetophenon-oximethern der allgemeinen Formel

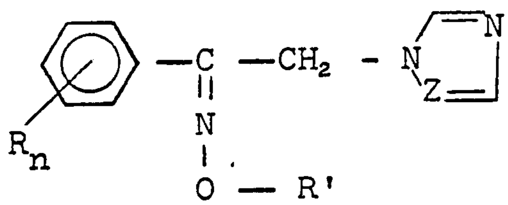
(IV)

in welcher R, R' und n die in Anspruch 1 angegebene Bedeutung besitzen und Z für die CH-Gruppe oder ein Stickstoffatom steht, dadurch gekennzeichnet, daß man die ω-Halogen-acetophenon-oximether der Formel I in Anspruch 1 mit Azolen der Formel

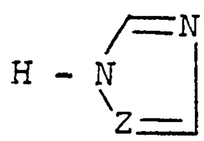
(V)

in welcher Z die obenangegebene Bedeutung hat, in Gegenwart eines Säurebindemittels und in Gegenwart eines Verdünnungsmittel umsetzt.

**Claims**

1. ω-Halogeno-acetophenone oxime ethers of the general formula

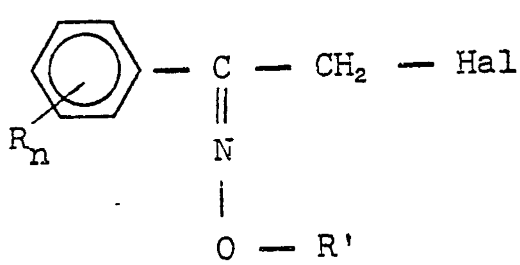
(I)

in which

R represents fluorine, chlorine, bromine, nitro and cyano, further represents alkyl and alkylsulphonyl with in either case 1 to 4 carbon atoms, alkoxy and alkylthio with 1 to 2 carbon atoms and halogenoalkyl

with up to 2 carbon atoms and up to 3 halogen atoms, and in addition represents phenyl or phenoxy, it being possible for these two radicals to be substituted by halogen, cyano, nitro and halogenoalkyl with up to 2 carbon atoms and up to 3 halogen atoms,

R' represents alkyl, alkenyl and alkynyl with in each case up to 4 carbon atoms and cycloalkylalkyl with 5 to 7 carbon atoms in the cycloalkyl part and 1 to 2 carbon atoms in the alkyl part, it being possible for the cycloalkyl part to be optionally substituted by alkyl with 1 to 4 carbon atoms, and represents benzyl or styryl, it being possible for the indicated two radicals to be substituted by halogen, cyano, nitro, amino, alkyl with 1 to 4 carbon atoms, phenyl, phenoxy and halogenoalkyl with up to 2 carbon atoms and up to 3 halogen atoms,

n represents the numbers 1 or 2 and

Hal represents chlorine or bromine.

2. Process for the preparation of ω-halogeno-acetophenone oxime ethers of the formula I in Claim 1, characterised in that ω-halogeno-acetophenones of the formula

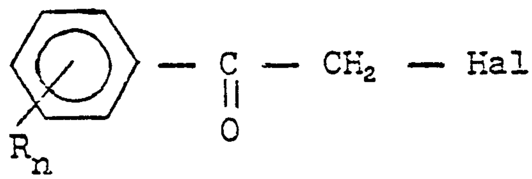

(II)

in which

Hal, R and n have the meaning given in Claim 1, are reacted with substituted hydroxylamines of the formula

$H_2N—O—R'$ (III)

in which

R' has the meaning given in Claim 1, in the presence of a diluent.

3. Use of ω-halogeno-acetophenone oxime ethers of the formula I in Claim 1 as intermediate products for the preparation of largely known ω-azolyl-acetophenone oxime ethers of the general formula

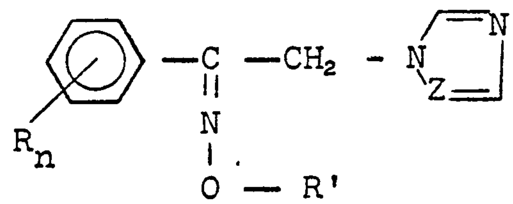

(IV)

in which R, R' and n have the meaning given in Claim 1 and Z represents the CH group or a nitrogen atom, characterised in that the ω-halogeno-acetophenone oxime ethers of the formula I in Claim 1 are reacted with azoles of the formula

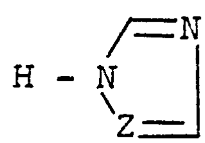

(V)

in which Z has the abovementioned meaning, in the presence of an acid-binding agent and in the presence of a diluent.

**Revendications**

1. Ethers d'oximes d'ω-halogéno-acétophénones de formule générale :

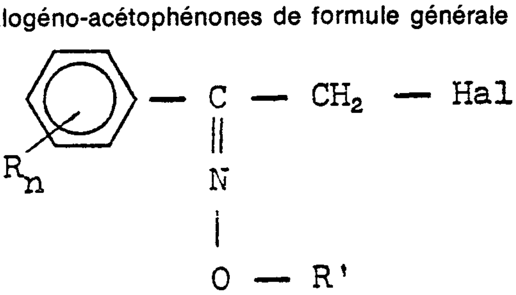

(I)

dans laquelle

R est le fluor, le chlore, le brome, le groupe nitro et le groupe cyano, en outre un groupe alkyle et un groupe alkylsulfonyle ayant chacun 1 à 4 atomes de carbone, un groupe alkoxy et un groupe alkylthio ayant 1 ou 2 atomes de carbone ainsi qu'un groupe halogénalkyle ayant jusqu'à 2 atomes de carbone et jusqu'à 3 atomes d'halogènes, en outre le groupe phényle ou le groupe phénoxy, ces deux restes pouvant être substitués par un halogène, un groupe cyano, un groupe nitro ainsi qu'un groupe halogénalkyle ayant jusqu'à 2 atomes de carbone et jusqu'à 3 atomes d'halogènes,

R' est un groupe alkyle, un groupe alcényle et un groupe alcynyle ayant chacun jusqu'à 4 atomes de carbone et un groupe cycloalkylalkyle ayant 5 à 7 atomes de carbone dans la partie cycloalkylique et 1 ou 2 atomes de carbone dans la partie alkylique, la partie cycloalkylique pouvant être substituée, le cas échéant, par un radical alkyle ayant 1 à 4 atomes de carbone, ainsi que le groupe benzyle ou le groupe styryle, les deux restes mentionnés en dernier lieu pouvant être substitués par un halogène, un groupe cyano, nitro, amino, alkyle ayant 1 à 4 atomes de carbone, le groupe phényle, le groupe phénoxy ou un groupe halogénalkyle ayant jusqu'à 2 atomes de carbone et jusqu'à 3 atomes d'halogènes,

n représente les nombres 1 ou 2 et

Hal est le chlore ou le brome.

2. Procédé de production d'éthers d'oximes d'ω-halogéno-acétophénones de formule I suivant la revendication 1, caractérisé en ce qu'on fait réagir des ω-halogéno-acétophénones de formule :

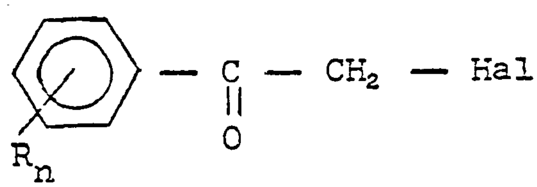

(II)

dans laquelle

Hal, R et n ont la définition indiquée dans la revendication 1, avec des hydroxylamines substituées de formule :

$$H_2N-O-R' \quad \text{(III)}$$

dans laquelle

R' a la définition indiquée dans la revendication 1, en présence d'un diluant.

3. Utilisation d'éthers d'oximes d'ω-halogéno-acétophénones de formule I suivant la revendication 1 comme produits intermédiaires pour la préparation d'éthers d'oximes d'ω-azolyl-acétophénones très connus de formule générale :

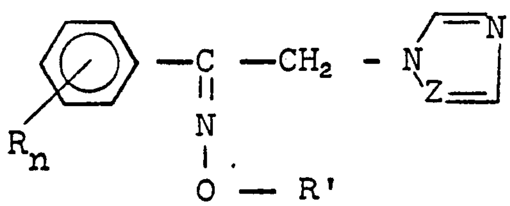

(IV)

dans laquelle R, R' et n ont la définition indiquée dans la revendication 1 et Z est le groupe CH ou un atome d'azote, caractérisée en ce qu'on fait réagir les éthers d'oximes d'ω-halogéno-acétophénones de formule I suivant la revendication 1 avec des azoles de formule :

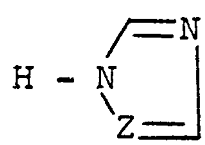

(V)

dans laquelle Z a la définition indiquée ci-dessus, en présence d'un accepteur d'acide et en présence d'un diluant.